# EUROPEAN PATENT SPECIFICATION

(11) **EP 0 602 086 B1**
(45) Date of publication and mention of the grant of the patent: **28.02.1996**
(21) Application number: 92918184.0
(22) Date of filing: 03.09.1992
(51) Int. Cl.: C07C 409/24, C07C 407/00, A01N 37/16, C11D 3/39

(54) **PREPARATION OF PEROXYACIDS**
VERFAHREN ZUR HERSTELLUNG VON PERSÄUREN
PREPARATION DE PEROXYACIDES

(30) Priority: 04.09.1991 GB 9118906
(43) Date of publication of application: 22.06.1994
(73) Proprietor: SOLVAY INTEROX LIMITED, Warrington, Cheshire WA4 6HB (GB)
(72) Inventor: WILSON, Sharon, Lesley, Warrington Cheshire WA5 5YB (GB); CARR, Graham, Liverpool L25 5HR (GB)
(74) Representative: Pearce, Timothy
(86) International application number: GB9201609
(87) International publication number: WO9305016

(56) References cited:
- EP-A- 0 024 219
- WO-A-91/13058

## Description

The present invention relates to a process for the preparation of peroxyacids, and in particular to a process which is suitable for the preparation of hydroxyaliphatic peroxyacids and to dilute aqueous compositions containing them. In a further aspect, the present invention relates to the use of such peroxyacids and compositions for disinfection.

Many disinfectant solutions aimed at personal, domestic, industrial or health markets have been based in the past upon active chlorine compounds. Doubts have been expressed about the safety of using chlorinaceous compounds in view of the possible generation of carcinogenic or otherwise harmful substances, albeit in very low or trace amounts. In consequence, efforts have been made to locate alternative, non-chlorinaceous disinfectants.

It has hitherto been proposed to employ dilute solutions of peroxyacetic acid or like compounds, eg peroxypropanoic acids as disinfectants. Solutions of such compounds can be employed effectively as disinfectants in a number of applications, but some people find the odour of, in particular, peroxyacetic acid solutions offensive or irritating. Consequently, for applications in which the disinfectant is likely to be employed in the proximity of people, it remains desirable to try to find alternative disinfectants.

In the course of the investigations leading to the present invention, a range of peroxyacids was found that was free from olfactory or irritational offence, or at least was offensive to only a lesser extent. These compounds are encompassed by the term low molecular weight hydroxyaliphatic peroxyacids. Dilute solutions containing such hydroxyaliphatic peroxyacids can be made by a variety of methods employing reaction between hydrogen peroxide and the precursor hydroxyaliphatic carboxylic acid. It has been found that the efficiency of conversion of hydrogen peroxide to peroxyacid species in the course of producing dilute aqueous solutions of the hydroxyaliphatic peroxyacids and/or the peroxyacid stability of the dilute compositions during storage prior to use varies from method to method. It will be recognised that peroxyacid species are more effective than hydrogen peroxide as disinfectants so that it is advantageous to devise manufacturing methods which increase the efficiency of conversion. Likewise, the stability of the solutions, and in particular the stability of the peroxyacid species in solution is of practical importance in that it governs its effective useful life as a disinfectant.

Certain hydroxyaliphatic carboxylic acids, such as citric acid and tartaric acid in particular, have been suggested as stabilisers for hydrogen peroxide solutions, as for example in EP-A-0 233 350 to Henkel or incorporated in special stabiliser solutions for addition with hydrogen peroxide solutions in water treatments, such as in PCT Application published as WO 91/8981 to Aquaclear or in GB-A-2 189 394 to Sanosil. Alternatively, such carboxylic acids have been proposed for acidification of alkaline solutions as in GB-A-2 169 308 to Kao Corp, or as a complexing agent in alkaline bleaching solutions as for example in EP-A-0 157 483 to Interox Chemicals Limited. However, none of those references disclose processes for improving the efficiency of production of peroxyacid species as contemplated in the present invention.

According to the present invention there is provided a process for the formation of dilute aqueous solutions of an hydroxyaliphatic peroxycarboxylic acid preferably containing not more than 7 carbons in which an hydroxy-aliphatic carboxylic acid is contacted with hydrogen peroxide solution, characterised in that in a first step a concentrated aqueous solution of the hydroxy-aliphatic carboxylic acid is mixed with a concentrated hydrogen peroxide solution containing at least 0.5 moles hydrogen peroxide per molar equivalent of hydroxyaliphatic carboxylic acid in the presence, if necessary, of a catalytic amount of a strong acid, in a second step the mixture is stored until the concentration of peroxyacid has closely approached its maximum, and in a third step, the mixture is diluted by the introduction of water, optionally containing additional hydrogen peroxide and/or hydroxyaliphatic carboxylic acid.

Advantageously, the selection of hydroxyaliphatic peroxycarboxylic acids produced by a process of the present invention has been found to possess desirable qualities, namely the retention of an ability to act as a disinfectant whilst being undetectable or virtually undetectable olfactorily by humans.

The presence of the hydroxyl group within the molecule renders the peroxyacid particularly soluble.

It has been found in a number of instances that by employing a process according to the present invention to make dilute compositions, the conversion of hydrogen peroxide to peroxycarboxylic acid is greater than if the same amounts of constituents, viz hydroxycarboxylic acid and hydrogen peroxide are mixed together in dilute solution. In at least some of the foregoing instances or in other comparisons, it has additionally or alternatively been found that the resultant dilute composition produced by the method of the present invention showed a demonstrable decrease in the rate of decomposition of the peroxyacid species.

In the present invention, the hydroxy-carboxylic acid which is peroxidised may be a monocarboxylic acid, or preferably is either a dicarboxylic acid or a tricarboxylic acid. Examples of these three variations include, respectively, lactic acid, tartaric or malic acid and citric acid. Many of the aliphatic acids employed herein contain a single hydroxyl group or two hydroxyl groups, and in most instances contain a carboxylic acid to hydroxyl ratio of not greater than 1:1.

Without being bound or circumscribed by any theory or proposed explanation, it has been postulated that the difference in efficiency of conversion to peroxyacid species and/or difference in stability of the resultant dilute solutions for compounds containing a plurality of carboxylic acid groups may arise, at least in part from the extent to which a plurality of the carboxylic acid groups are oxidised in each molecule or conversely the extent to which only a single carboxylic acid group is oxidised. In other words, the different methods for producing dilute solutions, it is believed, may be producing compositions containing a different balance of peroxyacid species even though they are produced using the same ratio of hydrogen peroxide to hydroxyaliphatic carboxylic acid and in the presence finally of the same amount of diluent water.

The concentration of hydroxyaliphatic carboxylic acid in the solution employed in step 1 of the instant process, preferably comprises at 30% by weight and in a number of instances advantageously is around 50% or more, on the same basis. It is often desirable to employ a saturated or near saturated solution of the carboxylic acid, such as for example a concentration of at least 9/l0ths that of a saturated solution at the selected temperature. Employment of a saturated or near saturated solution is particularly desirable when employing hydroxyaliphatic carboxylic acids which are soluble to the extent of less than 50% by weight at saturation.

The saturated or near saturated aqueous solution of the hydroxyaliphatic acid is thereafter mixed with a concentrated aqueous hydrogen peroxide solution, preferably containing at least 50% w/w hydrogen peroxide and especially at least 70%. It is particularly desirable to introduce hydrogen peroxide solutions which have a concentration in the range of from 75 to 90% w/w hydrogen peroxide. In many instances, it is desirable to employ at least 0.5 moles of hydrogen peroxide per mole equivalent of carboxylic acid group, and often up to an equivalent mole ratio of 1:1. It will be recognised that at least when a plurality of carboxylic acid groups is present in the hydroxyaliphatic acid, the amount of hydrogen peroxide is greater than stoichiometric as regards peroxidising one carboxylic acid group but substoichiometric as regards peroxidising all of the carboxylic acid groups in the molecule. Results of particularly practical value have been obtained when the equivalent mole ratio of peroxide to carboxylic acid group is chosen in the range of from 0.55:1 to 0.8:1. Notwithstanding the foregoing, for the reason of avoiding potentially hazardous conditions, though, it is sensible to so select the reactants that the concentration of hydrogen peroxide in the carboxylic acid-containing reaction mixtures is not greater than about 20% w/w.

The reaction between the hydroxycarboxylic acid and the hydrogen peroxide is usually conducted in the presence of a strong acid, typically having a pKₐ of below 0, such as a mineral acid or an organic sulphonic acid which acts as a catalyst. Examples include sulphuric acid, phosphoric acid and methane sulphonic acid. In many instances the proportion of strong acid catalyst is chosen in the region of from 0.25 to 5% w/w of the reaction mixture, and in preferred instances from 0.75 to 2% w/w. By selecting such a comparatively high concentration of strong acid catalyst it is possible to enable the peroxidation to occur at a convenient rate without the use of elevated reaction temperatures.

The peroxidation reaction is usually conducted at a temperature of not greater than about 50°C and at not less than about 5°C. For convenience, coupled with safety considerations, the reaction temperature is maintained, in many embodiments in the range that is at or around ambient, viz from about 15 to about 30°C.

The reaction is permitted to continue until peroxidation of the carboxylic acid group or groups has occurred to the empirical maximum or at least a substantial fraction thereof. This can be achieved by monitoring the peracid content of the reaction mixture and the proportion of hydrogen peroxide still unreacted, by for example periodic withdrawal of a sample and analysis for both peracid available oxygen (often abbreviated to Avox ) and hydrogen peroxide Avox. As the peroxidation reaction nears its maximum, the rate of increase in peracid concentration tends to decelerate. From such monitoring, it can be gauged when peracid concentration has reached a maximum or a prediction can readily be made as to when that maximum will be reached. The monitoring can be conducted on a small-scale pilot trial and applied to larger scale working or may be carried out on the full scale process. As a further variation, when a number of trials have already been conducted, a restricted number of samples may be taken at periods which experience indicates would confirm that conversion of the carboxylic acid to percarboxylic acid is progressing to an expected extent, rather than fully following the conversion profile throughout the reaction.

By employing a suitable concentration of catalyst within the range identified hereinbefore, it is possible to attain a peracid concentration that is at or near a maximum in a period of less than 8 hours , and advantageously a period of not greater than 3 hours is often sufficient. The reaction period is usually at least 45 minutes, though if monitoring indicated that a shorter period was feasible for a particular embodiment, then the reaction may be terminated then.

The solution obtained during the peroxidation reaction is diluted by mixture with water and this has the effect of quenching the peroxidation reaction. Indeed thereafter there is some possibility of a slow loss of peracid by acid hydrolysis. The extent of dilution is at the discretion of the process operator. In many instances, at least an equivalent weight of diluent water is employed and for example up to about 5 times the weight. It is often convenient to dilute until the solution has a peracid concentration in the region of about 0.5 to 2% w/w, typically around 1%. A composition containing such a concentration of peracid represents a ready to use disinfectant solution, for example domestically.

It is desirable to effect the dilution soon after the point of maximum peracid concentration has been attained, the leeway depending to at least some extent on the rate at which the peracid concentration is changing. For a number of peracids, dilution often is effected within 20% of the elapsed reaction period. For example for tartaric acid, this can be 5 to 30 minutes or less after the point of maximum peracid concentration has been recognised.

It can be convenient to introduce further acid catalyst in the dilution water, although it is not essential to do so, though preferably not to exceed about 1% catalyst concentration.

The dilution stage is normally conducted at or around ambient temperature. The dilution water often has a temperature of from about 5 to 25°C.

At the dilution stage, it is often advantageous to introduce a further amount of hydrogen peroxide. In general the amount is not greater than about 1 mole per equivalent mole of carboxylic acid, and often the amount introduced at the dilution stage is such that together with the amount employed in the peroxidation stage, the combined amount is commonly up to about 1.2 moles hydrogen peroxide per equivalent mole of carboxylic acid group, and usually at least 0.6 moles per equivalent mole. In a number of instances the amount of hydrogen peroxide introduced in the dilution stage is from a quarter to two thirds the amount employed in the peroxidation reaction. It will be recognised therefore that in such advantageous embodiments, the invention process provides a superior and more effective way of employing a given amount of hydrogen peroxide that is in the general region of a stoichiometric amount. This is of course a different concept from the use of a vast excess of hydrogen peroxide, concentrations of the latter of at least 25% contemplated in USP 4 051 058 and USP 4 051 059 to Bowing and concentrations of over 30% hydrogen peroxide contemplated by Crommelynck in USP 4 297 298.

It will be understood that an additional amount of the hydroxycarboxylic acid can also be introduced at the dilution stage. By so doing, usually in combination with added hydrogen peroxide, it is possible to produce dilute compositions containing the principal components, viz water, hydrogen peroxide, peracid species and hydroxycarboxylic acid in equilibrium or close thereto.

The combination in the instant invention of a reasonably fast peroxidation reaction using a saturated or near saturated solution of the hydroxy-carboxylic acid and dilution with a solution preferably containing a second and lesser amount of hydrogen peroxide has produced disinfectant solutions which retain their peracid concentrations longer, and hence their disinfectant effectiveness longer, than if simple dilute solutions were made using similar amounts of reagents. The concentration of hydrogen peroxide solution employed at the dilution step can be broader than in the peroxidation step. In general, peroxide concentrations of at least 6% w/w are employable and the water content therein contributing in a readily calculable fashion to the overall dilution.

Additionally, the dilute peracid compositions can contain, if desired, a chelating agent for metals and particularly transition metals which catalyse decomposition of peroxy species in aqueous solution, including both hydrogen peroxide and peracid species. Suitable chelating agents are often aminopolycarboxylic acids or salts thereof such as EDTA or DTPA, and/or carboxylic acid substituted N-containing heterocyclics, such as 8-hydroxyquinoline or picolinic or dipicolinic acid, and organopolyphosphonates, including hydroxyethylidenediphosphonic acid, and alkyleneaminomethylene (phosphonic acid)s such as ethylene diaminotetra methylene phosphonic acid, cyclohexane-1,2-diaminotetra methylene phosphonic acid and diethylenetriaminepenta methylene phosphonic acid. A combination of an organophosphonate and an N-heterocyclic carboxylic acid is particularly suitable. The amount of chelant in the composition is at the discretion of the formulator, but is preferably greater than 0.25% and often not greater than about 1.5%, calculated as active material therein.

It will be recognised that the essence of the present invention resides in the process operations identified hereinabove and in particular the production of a more concentrated solution and the point in time at which it is diluted. This process has been described with particular reference to the formation of peracid compositions from hydroxyaliphatic carboxylic acids.

The compositions produced by the process according to the present invention can conveniently be employed for disinfection without adjustment to their pH, that is to say at the moderately acidic pH which they enjoy. Their effectiveness is often best in the region of about pH 2.5 to about pH 5.

The compositions made by the invention process can be employed to treat a wide range of substrates. Many of the treatable substrates are either liquid or solid. A contaminated gaseous substrate can be treated conveniently by spraying with a dilute solution of the invention biocidal combination or by bubbling the gas through a bath of the invention peracid solution. One type of liquid substrate comprises micro-organism contaminated aqueous media such as recirculating process waters, or aqueous effluents prior to discharge. Such process waters occur in many different industries and can be contaminated by bacteria, algae, yeasts and more rarely by viruses. Without limiting to the following industries, contaminated process waters are prevalent during the processing of plant and animal materials, including the paper and pulp industries, the sugar refining industry, brewing, winemaking and alcohol distillation industries, effluents from straw treatments, discharges from sewage treatment works, including partially treated or merely filtered discharges of sewage through pipelines extending out to sea, meat processing factories, carcass rendering activities and from the rearing of livestock. A further important source of contaminated aqueous media comprises cooling waters either industrially or arising from air conditioning units installed in large buildings, such as hotels, offices and hospitals.

The invention compositions can be employed to treat non-aqueous liquid media, such as cutting oils.

Notwithstanding the foregoing, the invention compositions are seen as of particular value for disinfection in those areas which come into contact with humankind. Thus they can be employed to disinfect solids, including hard surfaces, or contaminated articles intended for re-use in the food-rearing, horticulture, domestic or hospital environments. Hard surfaces can be made from metals, wood, ceramics, glass, and plastics and can include work-benches, walls, floors, sanitaryware, plant or apparatus, containers, tools, machinery, plant and pipework. It will be recognised that for such hard surfaces, it is often convenient to immerse smaller articles in a solution of the invention biocidal composition, and for larger applications, a spray or the like distribution means can be easier to employ. The process can also be contemplated for disinfecting water absorbent materials such as infected linen or especially soiled babies' nappies that are often made from terry towelling.

The invention compositions can be used to disinfect harvested plants or plant products including seeds, corms, tubers, fruit, and vegetables. Alternatively, typically after further dilution the invention compositions can be used to treat growing plants, and especially crop growing plants, including cereals, leaf vegetables and salad crops, root vegetables, legumes, berried fruits citrus fruits and hard fruits, by virtue of the nature of the peracid itself and the hydroxy-carboxylic acid from which it is derived.

It will none the less also be recognised that the peracid solutions produced by the invention process may also be employed, if desired, for the other purposes for which peracids are used, including bleaching or as a bleach additive in washing processes.

Having described the invention in general terms, specific and non-limiting embodiments thereof will hereinafter be described in greater detail by way of example only.

### Comparisons A to C and Examples 1 to 3

In comparisons A to C,aqueous dilute solutions were made from hydroxycarboxylic acids and hydrogen peroxide by mixing all the constituents together at ambient temperature, about 23°C in a glass vessel, the method being similar to that described in European Patent Specification 00 13 886A. The constituents comprised demineralised water, 50g, into which was dissolved with stirring hydroxyaliphatic carboxylic acid, 10g, respectively tartaric, malic or lactic, conc sulphuric acid, 0.5g, aqueous hydrogen peroxide solution, (85% w/w, 6.25g) and as stabiliser, 0.5g of a solution of hydroxy ethylidene-l,l,diphosphonic acid as supplied under the Trade Mark DEQUEST 2010 and dipicolinic acid, 0.1g.

In Examples 1 to 3, which are according to the present invention, dilute peracid compositions were made by dissolving 10g of respectively, tartaric acid, malic acid or lactic acid in demineralised water, 7.8g, thereby forming a substantially saturated solution. Concentrated sulphuric acid (98% w/w, 0.2g) and then aqueous hydrogen peroxide solution (85% w/w, 4.0g) was introduced with stirring over a few minutes at ambient temperature, about 23°C. The peracid content of the reaction mixture was monitored and after about 1 hour, it was found that the rate of increase in peracid concentration was barely detectable and the solution was then diluted by introduction of demineralised water, 42.2g, aqueous hydrogen peroxide solution, (85%, 2.25g) and conc sulphuric acid, 0.3g. The same stabilisers, DEQUEST 2010, 0.5g and dipicolinic acid, 0.1g were also incorporated. The total amount of water, carboxylic acid, hydrogen peroxide, sulphuric acid and stabiliser was the same in the comparisons and Examples.

The solutions produced in comparisons A to C and Examples 1 to 3 were then each stored at ambient temperature in polyethylene sample storage bottles, and their peracid Avox measured at intervals. The concentration of peracid Avox in the samples after 6 weeks storage, is indicated below:

| Peracid composition | | %Avox | Ratio of Ex/Comp |
|---|---|---|---|
| Tartaric | CompA | 0.13 | - |
| Tartaric | Ex1 | 0.18 | 1.38 |
| Malic | CompB | 0.07 | - |
| Malic | Ex2 | 0.23 | 3.28 |
| Lactic | CompC | 0.09 | - |
| Lactic | Ex3 | 0.20 | 2.22 |

From the data given above, it can be seen that the peracid stability of the dilute solutions is detectably and significantly greater when the solution are produced by the invention process than if the same constituents are mixed together in the same proportions in a single step. Thus, the invention process produces products having a significantly longer shelf life.

The microbiocidal capability of the compositions produced in the Examples was tested against Pseudomonas aeruginosa and Staphylococcus aureus, employing the method described by M G C Baldry in the Journal of Applied Bacteriology 1983, vol 54, pp417-423, contact time 5 minutes at 20°C. At a peracid concentration of only 10 ppm, at pH 3 and in unbuffered aqueous solution, all three peracids reduced the concentrations of the two standard bacteria by 10⁵.

### Comparison D and Example 4

In comparison D a dilute solution was made containing 7.3% citric acid, 15.5% hydrogen peroxide, and 1.5% conc sulphuric acid, (all %s w/w) and the balance demineralised water. In Example 4, a saturated citric acid solution was mixed with concentrated hydrogen peroxide solution, the mixture containing 47% citric acid, 16% hydrogen peroxide, 1% sulphuric acid and 1% stabiliser solution DEQUEST 2010 as supplied, permitted to attain maximum peracid Avox concentration and then diluted approximately 10 fold with demineralised water, to provide a dilute solution containing approximately the same concentration of peracid avox as produced in Comparison D. The concentrations of both citric acid and hydrogen peroxide were then brought up to their respective concentrations in Comparison D and stored at ambient temperature in the same way as for the preceding Comparison and Example solutions. Analysis of the stored solutions confirmed that the Example solution maintained its peracid avox better, exhibiting a slower loss of peracid in time, but in addition, the rate of loss of hydrogen peroxide was also markedly lower, approximately a quarter being lost from Comp D but only a tenth from Example 4.

## Claims

1. A process for the formation of dilute aqueous solutions of an hydroxyaliphatic peroxycarboxylic acid preferably containing not more than 7 carbons in which an hydroxy-aliphatic carboxylic acid is contacted with hydrogen peroxide solution, characterised in that in a first step a concentrated aqueous solution of the hydroxy-aliphatic carboxylic acid is mixed with a concentrated hydrogen peroxide solution containing at least 0.5 moles hydrogen peroxide per mole of hydroxyaliphatic carboxylic acid in the presence, if necessary, of a catalytic amount of a strong acid, in a second step the mixture is stored until the concentration of peroxyacid has closely approached its maximum, and in a third step, the mixture is diluted by the introduction of water, optionally containing additional hydrogen peroxide and/or hydroxyaliphatic carboxylic acid.

2. A process according to claim 1 characterised in that the hydroxyaliphatic carboxylic acid is a mono, di or tricarboxylic acid.

3. A process according to either of claims 1 or 2 characterised in that the acid is selected from lactic, malic, tartaric or citric acids.

4. A process according to any preceding claim characterised in that the solution employed in step 1 contains hydroxyaliphatic acid in a concentration of at least 30% w/w.

5. A process according to any preceding claim characterised in that at least 50% w/w hydrogen peroxide solution is introduced in step 1.

6. A process according to any preceding claim characterised in that at least 1 mole of hydrogen peroxide per mole of hydroxyaliphatic carboxylic acid is employed.

7. A process according to any preceding claim characterised in that when the hydroxyaliphatic acid molecule contains a plurality of carboxylic acid groups, the amount of hydrogen peroxide is greater than 1 mole per mole of the hydroxyaliphatic acid and less than 1 mole per equivalent mole of carboxylic acid group.

8. A process according to any preceding claim characterised in that the reaction period in which maximum peracid concentration is attained is from 45 minutes to 3 hours.

9. A process according to any preceding claim characterised in that the reaction mixture is diluted to produce a solution containing from 0.5 to 2% w/w peracid.

10. A process according to any one of claims 1 to 6, 8 or 9 characterised in that in the dilution stage, the hydrogen peroxide is introduced in such an amount that the combined amount with that introduced in the reaction step is up to 1.2 moles per mole of carboxylic acid group.

11. A process according to any preceding claim characterised in that the diluted solution contains a carboxylic acid and/or organophosphonic acid chelant.

12. Use of a dilute solution of a peroxyacid produced by a process according to any preceding claim as a disinfectant, preferably at a pH of from 2.5 to pH5.

## Patentansprüche

1. Ein Verfahren zur Bildung verdünnter wäßriger Lösungen einer hydroxyaliphatischen Peroxycarbonsäure, die vorzugsweise nicht mehr als 7 Kohlenstoffatome enthält, in welchem eine hydroxyaliphatische Carbonsäure mit einer Wasserstoffperoxidlösung kontaktiert wird, dadurch gekennzeichnet, daß in einer ersten Stufe eine konzentrierte wäßrige Lösung der hydroxyaliphatischen Carbonsäure mit einer konzentrierten Wasserstoffperoxidlösung, die mindestens 0,5 Mol Wasserstoffperoxid je Mol hydroxyaliphatischer Carbonsäure enthält, in Gegenwart, falls notwendig, einer katalytischen Menge einer starken Säure vermischt wird, daß in einer zweiten Stufe die Mischung gelagert wird, bis die Konzentration von Peroxysäure sich ihrem Maximalwert angenähert hat, und daß in einer dritten Stufe die Mischung durch Einführen von Wasser, das gegebenenfalls zusätzliches Wasserstoffperoxid und/oder hydroxyaliphatische Carbonsäure enthält, verdünnt wird.

2. Ein Verfahren nach Anspruch 1, dadurch gekennzeichnet, daß die hydroxyaliphatische Carbonsäure eine Mono- Di- oder Tricarbonsäure ist.

3. Ein Verfahren nach Anspruch 1 oder 2 ,dadurch gekennzeichnet, daß die Säure ausgewählt ist aus Milch-, Apfel-, Wein- oder Zitronensäure.

4. Ein Verfahren nach einem der vorstehenden Ansprüche, dadurch gekennzeichnet, daß die in Stufe 1 eingesetzte Lösung hydroxyaliphatische Säure in einer Konzentration von mindestens 30 Gewichtsprozent enthält.

5. Ein Verfahren nach einem der vorstehenden Ansprüche, dadurch gekennzeichnet, daß mindestens 50-gewichtsprozentige Wasserstoffperoxidlösung in Stufe 1 eingeführt wird.

6. Ein Verfahren nach einem der vorstehenden Ansprüche, dadurch gekennzeichnet, daß mindestens 1 Mol Wasserstoffperoxid je Mol hydroxyaliphatischer Carbonsäure eingesetzt wird.

7. Ein Verfahren nach einem der vorstehenden Ansprüche, dadurch gekennzeichnet, daß, wenn das hydroxyaliphatische Säuremolekül mehrere Carbonsäuregruppen enthält, die Menge an Wasserstoffperoxid größer als 1 Mol je Mol der hydroxyaliphatischen Säure und kleiner als 1 Mol je Moläquivalent der Carbonsäuregruppe ist.

8. Ein Verfahren nach einem der vorstehenden Ansprüche, dadurch gekennzeichnet, daß die Reaktionszeit, in welcher die maximale Persäurekonzentration erreicht wird, 45 Minuten bis 3 Stunden beträgt.

9. Ein Verfahren nach einem der vorstehenden Ansprüche, dadurch gekennzeichnet, daß die Reaktionsmischung zwecks Herstellung einer Lösung, enthaltend 0,5 bis 2 Gewichtsprozent Persäure, verdünnt wird.

10. Ein Verfahren nach einem der Ansprüche 1 bis 6, 8 oder 9, dadurch gekennzeichnet, daß in der Verdünnungsstufe das Wasserstoffperoxid in einer solchen Menge eingeführt wird, daß die kombinierte Menge, d.h. die Menge in Kombination mit der Menge, die in der Reaktionsstufe eingeführt worden ist, bis zu 1,2 Mol je Mol der Carbonsäuregruppe beträgt.

11. Ein Verfahren nach einem der vorstehenden Ansprüche, dadurch gekennzeichnet, daß die verdünnte Lösung eine Carbonsäure und/oder einen Organophosphonsäurechelatbildner enthält.

12. Verwendung einer verdünnten Lösung einer Peroxysäure, die durch ein Verfahren nach einem der vorstehenden Ansprüche hergestellt worden ist, als Desinfektionsmittel, vorzugsweise bei einem pH-Wert von 2,5 bis 5.

## Revendications

1. Procédé pour la formation de solutions aqueuses diluées d'acide peroxycarboxylique hydroxyaliphatique ne contenant de préférence pas plus de 7 atomes de carbone, dans lequel un acide carboxylique hydroxyaliphatique est mis en contact avec une solution de peroxyde d'hydrogène, caractérisé en ce que, dans une première étape, une solution aqueuse concentrée d'acide carboxylique hydroxyaliphatique est mélangée à une solution concentrée de peroxyde d'hydrogène contenant au moins 0,5 mole de peroxyde d'hydrogène par mole d'acide carboxylique hydroxyaliphatique en présence, si nécessaire, d'une quantité catalytique d'un acide fort, dans une deuxième étape, le mélange est stocké jusqu'à ce que la concentration en peroxyacide ait approché de près son maximum, et dans une troisième étape, le mélange est dilué par l'introduction d'eau, contenant facultativement du peroxyde d'hydrogène supplémentaire et/ou de l'acide carboxylique hydroxyaliphatique supplémentaire.

2. Procédé selon la revendication 1, caractérisé en ce que l'acide carboxylique hydroxyaliphatique est un acide mono-, di- ou tricarboxylique.

3. Procédé selon l'une quelconque des revendications 1 ou 2, caractérisé en ce que l'acide est choisi parmi les acides lactique, malique, tartrique ou citrique.

4. Procédé selon l'une quelconque des revendications précédentes, caractérisé en ce que la solution employée à l'étape 1 contient l'acide hydroxyaliphatique en une concentration d'au moins 30 % en poids (w/w).

5. Procédé selon l'une quelconque des revendications précédentes, caractérisé en ce qu'une solution de peroxyde d'hydrogène d'au moins 50 % en poids (w/w) est introduite à l'étape 1.

6. Procédé selon l'une quelconque des revendications précédentes, caractérisé en ce qu'au moins une mole de peroxyde d'hydrogène par mole d'acide carboxylique hydroxyaliphatique est employée.

7. Procédé selon l'une quelconque des revendications précédentes, caractérisé en ce que, lorsque la molécule d'acide hydroxyaliphatique contient une multitude de groupes d'acides carboxyliques, la quantité de peroxyde d'hydrogène est supérieure à 1 mole par mole d'acide hydroxyaliphatique et inférieure à 1 mole par équivalent molaire de groupes d'acides carboxyliques.

8. Procédé selon l'une quelconque des revendications précédentes, caractérisé en ce que la période de réaction dans laquelle la concentration maximum en peracide est atteinte, vaut de 45 minutes à 3 heures.

9. Procédé selon l'une quelconque des revendications précédentes, caractérisé en ce que le mélange réactionnel est dilué de façon à produire une solution contenant de 0,5 à 2 % en poids (w/w) de peracide.

10. Procédé selon l'une quelconque des revendications 1 à 6 , 8 ou 9, caractérisé en ce qu'à l'étape de dilution, le peroxyde d'hydrogène est introduit en une quantité telle que la quantité combinée à celle introduite à l'étape de réaction, vaut jusqu'à 1,2 moles par mole de groupes d'acides carboxyliques.

11. Procédé selon l'une quelconque des revendications précédentes, caractérisé en ce que la solution diluée contient un agent chélatant de type acide carboxylique et/ou de type acide organophosphonique.

12. Utilisation d'une solution diluée d'un peroxyacide produite par un procédé conforme à l'une quelconque des revendications précédentes en tant qu'agent désinfectant, de préférence à un pH de 2,5 à pH 5.
